# EUROPEAN PATENT APPLICATION

(11) **EP 4 437 934 A1**
(43) Date of publication of application: **02.10.2024**
(21) Application number: 23164406.3
(22) Date of filing: 27.03.2023
(51) Int. Cl.: A61B 3/00, A61B 3/10

(54) **IMAGING SYSTEM**

(71) Applicant: Leica Microsystems Inc., Deerfield, IL 60015 (US)
(72) Inventor: GHOFRANI, Mehran, Cary NC, 27519 (US)
(74) Representative: Zacco UK Ltd

(57) **Abstract**

A system for imaging a sample is disclosed. The system comprises a light source, a movable mirror, a photodetector, a first control unit and a second control unit. The light source generating a light beam and directing the light beam to the movable mirror which then deflects the light beam onto the sample. The photodetector detects light reflected from the sample. The first control unit steers the movable mirror according to position coordinates. The second control unit generates a data packet comprising the position coordinates and sending the data packet to the first control unit. The second control unit also generates trigger signals for the photodetector, the trigger signals being configured to trigger the photodetector to capture images corresponding to each of the position coordinates of the movable mirror. The first control unit and the second control unit communicate over Ethernet such that synchronization between the steering of the movable mirror and the triggering of the photodetector is enabled.

## Description

### TECHNICAL FIELD

The present disclosure relates to a system for imaging a sample. More specifically, the disclosure relates to a system for imaging a sample as defined in the introductory part of claim 1.

### BACKGROUND ART

An imaging system typically comprises complex optics and electronics which are often arranged at separated locations. Two-dimensional (2D) and three-dimensional (3D) imaging generally require movable mirrors in order to scan a sample. Movements of the mirrors need to be synchronized with a photodetector capturing a plurality of images. Such synchronization is often performed by multiple control units. Typically, these control units are synchronized through an analog drive system or a digital hardware. However, when the control units that require synchronization are not collocated, their synchronization becomes cumbersome.

Optical coherence tomography (OCT) is an imaging modality that uses principles of low coherence interferometry to generate three-dimensional images of a sample by scanning the sample path beam across two-dimensional spatial locations of the sample target. Such scanning systems typically use two orthogonal galvanometer mirrors separated in space to address the desired two-dimensional spatial locations of the sample. In order to produce a flat tomographic image plane, the spatial separation of the galvanometer mirrors require relay optics to combine the pupil of each scanning mirror to a common image pupil. This results in large format, multi optical element scanner designs which are less desirable for intraoperative systems requiring a minimal sterile field volume.

OCT scanners are available for use in a variety of surgical procedures where depth information is advantageous. For surgical procedures on small areas, or where access to the tissue region being operated on is restricted, for example, when using OCT scanners during e.g. eye surgery, care should be taken of the size of the scanner or its part. In particular, it may not be possible to have the entire scanner close to the patient and the scanner may require a distributed design. For instance, it may only be a scanning head that may be arranged close to the patient while the control engine may be located remotely.

In order to produce a tomographic image with high precision, real time synchronization between the scanning head comprising movable mirrors and a camera capturing the image is required. Known systems are not capable of fulfilling this demand.

### SUMMARY STATEMENTS

The disclosed technology seeks to mitigate or obviate at least some of the above-mentioned limitations by using Ethernet connection between two control units of the system.

According to a first aspect, a system for imaging a sample is disclosed. The system comprises a light source, a movable mirror, a photodetector, a first control unit, and a second control unit. The light source is configured to generate a light beam and to direct the light beam to the movable mirror which is further configured to deflect the light beam onto the sample. The photodetector is configured to detect light reflected from the sample. The first control unit is communicatively connected to the movable mirror and configured to steer the movable mirror according to position coordinates. The position coordinates may pertain to a tilt angle of the movable mirror. The second control unit is configured to generate a data packet comprising the position coordinates and to send the generated data packet to the first control unit. The second control unit is further configured to generate trigger signals for the photodetector. The trigger signals are configured to trigger the photodetector to capture images corresponding to the position coordinates of the movable mirror. The first control unit and the second control unit are configured to communicate over Ethernet such that synchronization between the steering of the movable mirror and the triggering of the photodetector is enabled.

The system may generate at least one of 2D or 3D images of the sample. The sample may be a plant, biological tissue, such as eye tissue, skin tissue, etc. The sample may comprise different reflecting layers to be imaged.

The light source may be a monochromatic light source, or a broadband light source, the light source may be a laser, such as a monochromatic laser, such as a broadband laser, etc. The light source may comprise one or more separate lasers, such as one light source being a source for a primary beam, and the other light source being a light source for a secondary light beam. The light source is generating a light beam which may be split into two or more light beams by a coupler. One light beam is then directed to the movable mirror. The light beam may be guided towards the movable mirror through free space or through an optical fiber. For instance, the primary beam may be directed to the movable mirror to thereby form a probe beam directed to the sample. The secondary light beam may be directed to the movable mirror, or to another reflective surface, to thereby form a reference light beam.

A movable mirror may be a mirror with two or more degrees of freedom. The system may also comprise more movable mirrors or a system of movable mirrors and some other optical elements for steering the light beam. The movable mirror may be a micro-electromechanical system (MEMS). The movable mirror may be controlled by a galvanometer. The light beam from the light source is then further deflected onto the sample. In some implementations, when the sample is in-vivo tissue, the movable mirror may be arranged in an imaging head, in a scanning head, or in a microscope head which can reach an organism to be imaged. The light beam deflected from the mirror is typically guided to the sample in free space.

In the present context, the photodetector may refer to a single photodetector, an array of photodetectors, a camera, or similar. The sample to be imaged reflects light and the photodetector, by collecting the reflected light generates an electric signal which may be further processed in order to generate an image of the sample. The photodetector may detect light reflected from the sample together with light from the reference beam to thereby form an image. Light reflected from the sample may be directed directly to the photodetector, or alternatively back to the movable mirror which further may deflect the light to the photodetector.

The first control unit and the second unit may be configured to control different components of the imaging system. The first control unit and the second control unit may be implemented as microcontrollers. According to the first aspect, the movable mirror is controlled by the first control unit and thereby communicatively connected thereto. Typically, the connection between the controller and the mirror is electrical allowing for sending control signals from/to the first control unit to/from the movable mirror. In some embodiments, the first control unit may be configured to control a horizontal and vertical tilt position of the reflective surface of the movable mirror. Typically, the first control unit and the movable mirror are collocated. The first control unit and the movable mirror may be collocated in a first system unit. In some implementations, the first control unit and the movable mirror (or a set of movable mirrors) are arranged in a scan-head, the scan-head may be used for scanning an in-vivo organism, or a part of it. The scan-head may comprise other components for further control or feedback from the movable mirror(s).

In some embodiments, the first control unit may be configured to implement closed loop control of the position of the movable mirror using a mirror position feedback signal. Further optical and electrical components may form part of the closed loop control.

The second control unit may be collocated with the photodetector. In implementations in which the light beam is guided from the light source to the movable mirror by an optical fiber as well as between the photodetector and the movable mirror, all three components may be collocated in a second system unit. The second system unit may be located separately from the first system unit. The second system unit may further include a host computer that may be configured for monitoring and managing of the imaging system. The second control unit may be connected with the light source for synchronization. The second control unit may be connected to a reference arm, which may also form part of the second system unit.

The second control unit may comprise a predefined list with position coordinates and may then, send these position coordinates to the first control unit for controlling the movable mirror which move in accordance with the position coordinates from the predefined list. For each set of position coordinates, the movable mirror will deflect a light beam based on which, the photodetector will capture a corresponding image. The predefined list with position coordinates may be saved in a memory the second control unit. In some implementations, the second control unit may receive the position coordinates from elsewhere, e.g. from a host computer that may be managing the entire imaging system. The position coordinates may define the exact position of the mirror, and/or a tilt angle of the movable mirror, and/or a voltage applied to the mirror, or other parameters that influence steering of the light beam by the mirror.

In order to obtain a multi-dimensional image of the sample, a plurality of images are obtained and grouped together. These plurality of images comprise data about the mirror position and thereby steering of the mirror, typically arranged away from the photodetector. Steering of the movable mirror requires real-time position updates in order to remain in sync with the photodetector image acquisition. One of the key requisites for high quality imaging is to ensure synchronization and coordination between the movable mirror and the photodetector. According to the present disclosure, to enable synchronization between the mirror and the photodetector, the first control unit steering and positioning the movable mirror and the second control unit controlling the triggering of the photodetector and image capturing are configured to communicate over Ethernet.

The use of Ethernet is an attractive solution for remote beam steering as it provides a standard interface with the option of providing power to the first control unit over data lines using Power over Ethernet (PoE) feature. Additional option of using standard protocol such as Internet Protocol (IP) provides an off-the-shelf solution for controlling beam steering as well as transmitting other command and control features in the scan-head. The beam steering requires a deterministic and fast update rate to keep the steering mirror closely synchronized with the photodetector. By connecting the first and second control unit via Ethernet, real-time synchronization can be achieved. The second control unit is configured to generate the data packet comprising the position coordinates, which are then sent over Ethernet, to the first control unit. The second control unit may timely generate trigger signals to the photodetector to capture an image that corresponds to the movable mirror moved according to the position coordinates sent to the first control unit. Namely, the second control unit may generate trigger signals at determined times to the photodetector. The synchronization between the photodetector and the mirror is configurable and determined times when the position updates are sent from the second controller to the first controller can be adjusted based on a system latency to thereby achieve best image quality.

The data packet sent from the second control unit to the first control unit may comprise other data relevant for synchronization of the photodetector and steering of the movable mirrors, such as predetermined delays that may exist in the imaging system. The data packet may also comprise information about the light source and in particular about the light beam generated by the light source. By communicating the data packet with the position coordinates over Ethernet from the second control unit simultaneously with triggering the photodetector, a reliable coordination and synchronization between the mirror positioning and the photodetector triggering controlled also by the second control unit is ensured. In this way, the image obtained by the photodetector corresponds to the corresponding position coordinates of the movable mirror that may be encoded in the obtained image.

In some embodiments, the first control unit and the second control unit are further configured to exchange one or more command/control signals over Ethernet. The commands/control signals may being related to the system operation. The command and control signals may include optical actuator controls, mirror controller runtime parameters, mirror controller performance monitoring, firmware update data, auxiliary light source control, such as keratoscope, and controller fault and status monitoring. Ethernet allows for exchange of various types of data and therefore exchange of the control and command signals is also possible apart from the position coordinates data exchange. By enabling exchange of command and control signals between the first and the second control unit an additional layer of communication between the two entities is enabled further improving the operation of the imaging system with distributed units.

In some embodiments, the first control unit may comprise a first field-programmable gate array (FPGA). The second control unit may comprise a second FPGA. The first and second FPGAs may both comprise a position update module and a command module. The position-update module may be configured to process data related to the position coordinates and the trigger signals. The command module may be configured to process the commands/control signals. Alternatively, the first control unit and/or the second control unit may each comprise two FPGAs communicatively connected. Utilizing FPGAs in communication of a distributed imaging system is advantageous as FPGAs can be configured in accordance with demands of the imaging system.

In some embodiments, the Ethernet communication between the first and the second control unit is implemented over an Internet Protocol (IP), such as User Data Protocol (UDP), Transmission Control Protocol (TCP), or similar. UDP is a generic connection-less IP transport layer protocol standard that offers a low-overhead implementation option. Other protocols such as TCP, various UDP variants, or even a custom transport protocols can be used in some embodiments. Different protocols may have different demands in terms of processing power when handling an additional overhead or developing or ensuring that support exists in the TCP/IP software stack. Using an IP protocol, such as UDP ensures a real-time mirror position updates in the order of less than 10 psec.

In some embodiments, the second control unit comprises a scan-control block configured to generate the trigger signal to the photodetector and provide position coordinates to a traffic-control block. The traffic-control block may be configured for sending the data packet comprising the position coordinates and/or command/control requests to the first control unit. The scan-control block may fetch the predetermined position coordinates from a memory in the second system unit. A host computer, managing the imaging system, may be responsible for writing these predetermined position coordinates into the memory. The traffic controller, upon receiving the position coordinates from the scan-control block, may initiate Ethernet transfer of the position coordinates to the first controller. The traffic controller may first send the position coordinates to a direct memory access (DMA) controller for preparing the data packet, such as a UDP packet. The DMA controller may further initiate a DMA process to transfer the packet over Ethernet. The scan control block ensures that the triggering signal is synchronized with sending of the position coordinates to the first control unit. Furthermore, the traffic-control block may communicate both the triggering signal and the corresponding position coordinates to the host computer. These data may be used in forming the multi-dimensional image of the sample.

In some embodiments, the trigger signals are based on a predetermined photodetector frame rate. These trigger signals are typically generated by the scan-control block. The scan-control block may comprise a configurable timer configured to generate these triggers. The photodetector, e.g. a photodiode, a camera, or similar, is characterized by the predetermined frame rate and may be capable of detecting a signal and acquiring an image only at a predetermined time slots defined by the frame rate. As the trigger signals generation is closely related to the generation of the data packets comprising the position coordinates, generating the trigger signals on the basis of the photodetector frame rate implies that the steering of the movable mirror is also performed on the basis of the predetermined photodetector frame rate. In some implementations, the trigger signals based on the predetermined photodetector frame rate and generated by the scan-control block may also be sent to the traffic control block responsible for generating and sending the data packets with the position coordinates. The trigger signal may initiate sending of the data packet over Ethernet. By generating trigger signals on the basis of the photodetector frame rate it is ensured that the steering of the movable mirror is also performed on the basis of the photodetector frame rate. In this way, the movable mirror does not take positions that cannot be followed by the photodetector.

In some embodiments, the data packet further comprises the one or more commands/control signals. If there are commands or controls from the control module of the FPGA that need to be sent to the first control unit or to the first unit of the imaging system, e.g. the scan-head, a request may be issued to the traffic controller of the second control unit to forward the data packet to the scan-head with control/command signals interleaved with the position updates.

In an alternative embodiment, the one or more command/control signals may be placed a separate data packet, e.g. a command/control data packet, which may be sent separately from the data packet with the position coordinates. For instance, the command/control data packet may be sent in between the position update intervals. Sending of these data packet may not be related to the trigger signals and may not affect the position update timing. The control module of the second FPGA may initiate command/control forwarding by signalling to the traffic controller block. The command/control may be placed in a UDP control/command packet, and a memory address of the UDP control/command packet may be passed to the traffic controller. The traffic controller may follows similar sequence of steps to place the UDP control/command packet in the next available buffer descriptor for the Ethernet block to process.

In some embodiments, the scan-control block is further configured to modify the position coordinates on the basis of configuration parameters. Modification of the position coordinates may include offset, scaling, rotation, and/or distortion corrections. The configuration parameters may relate to a delay in communication between the first and second control unit, to various delays that may exist in the imaging system, or similar. Upon modifying the position coordinates, the scan-control block may then generate a position update request to the traffic-controller. By modifying the position coordinates on the basis of various system configuration parameters precision of the imaging is improved.

In some embodiments, the traffic-control block is configured to receive the modified position coordinates from the scan-control block and to update the data packet with the modified position coordinates. The traffic controller copies the new position coordinates into the data packet. The new position coordinates may be copies into a predefined UDP packet buffer and the traffic controller may update the UDP packet header fields accordingly. The traffic controller may then fetch a DMA tail link-list pointer and identify the next available buffer descriptor. Updates of the buffer descriptor with the pointer to the UDP packet may be followed by updating the Ethernet block tail buffer descriptor pointer. Once the Ethernet block tail buffer descriptor pointer is updated, the updated data packet may be transmitted to the first control unit.

In some embodiments, the first control unit, upon receiving the data packet with the position coordinates is configured to retrieve the position coordinates from the data packet and to steer the movable mirror accordingly. The received position coordinates may also be the updated/modified position coordinates. The first control unit, upon receiving the data packet or the control/command data packet may be configured to derive the one or more command/control signals for further processing. The first control unit may as well comprise its own traffic controller, the first traffic controller. The first traffic controller may evaluate the incoming packet header to first ensure the protocol type and header parameters (e.g. UDP header parameters). The first traffic controller may then filter out the position coordinates and place the new position coordinates in a digital controller designated memory position, the digital controller configured to control steering of the mirrors. Other commands/controls may be passed on to a microcontroller lightweight IP (LwIP) driver for upper software processing.

In some embodiments, the one or more command/control signals comprise a failsafe command configured to shut-down the light source if the first controller is not active or malfunctions and/or communication between the first and the second control unit is lost. The first control unit may send out periodic "scan-active" commands back to the second system module, or directly to the second control unit. If the "scan-active" fails to arrive within a certain time window, the shutdown timer may disable the light-source. In this way, imaging of the sample can be disabled if steering of the mirror is not functioning properly. In some embodiments, the photodetector may as well be disabled if the first controller is not active or malfunctioning.

According to a second aspect, a method for controlling a system for imaging a sample is disclosed. The system comprises components as discussed in connection with the first aspect, i.e. a light source, a movable mirror, a photodetector, a first control unit, and a second control unit. The light source is configured to generate a light beam and to direct the light beam, preferably via an optical fibre, to the movable mirror which is further configured to deflect the light beam onto the sample, preferably via free space. The photodetector is configured to detect light reflected from the sample. The first control unit is communicatively connected to the movable mirror, preferably electrically connected. The second control unit is communicatively connected to the photodetector. The first control unit and the second control unit are configured to communicate over Ethernet.

The method comprises the steps of: (a) generating, at the second control unit, a data packet comprising position coordinates, the position coordinates preferably pertaining to a tilt angle of the movable mirror, (b) sending, by the second control unit over Ethernet, the data packet to the first control unit, (c) steering, by the first control unit, the movable mirror according to the position coordinates, (d) generating, at the second control unit, trigger signals for the photodetector such that synchronization between the steering of the mirrors and the triggering of the photodetector is enabled, the trigger signals being configured to trigger the photodetector to capture images corresponding to the position coordinates of the movable mirror.

According to a third aspect, an optical coherence tomography scanner for imaging a sample is disclosed. The scanner comprises a light source, a movable mirror a photodetector, a first control unit, a second control unit, and an image processor. The light source is configured to generate a light beam and to direct the light beam to the movable mirror which is further configured to deflect the light beam onto the sample. The photodetector is configured to detect light reflected from the sample and to send the detected signal to the image processor configured to generate an image of the sample. The first control unit being communicatively connected to the movable mirror and configured to steer the movable mirror according to position coordinates, the position coordinates preferably pertaining to a tilt angle of the movable mirror. The second control unit is configured to generate a data packet comprising the position coordinates and sending the data packet to the first control unit and the second control unit being further configured to generate trigger signals for the photodetector, the trigger signals being configured to trigger the photodetector to capture images corresponding to the position coordinates of the movable mirror. The first control unit and the second control unit are configured to communicate over Ethernet such that synchronization between the steering of the movable mirror and the triggering of the photodetector is enabled.

The OCT scanner may further comprise a reference arm. A person skilled in the art is aware of use of the reference arm in the OCT scanner. The reference arm may be fiber-based and/or free-space based and typically guides a beam used for feedback on the movable mirror which is reflected in a different optical plane from that used by the OCT scanning beam.

It is beneficial to design an OCT scanner comprising two modules, i.e. a control module comprising the second control unit, and preferably, the light source, the photodetector, and the image processor, and a scan-head comprising the first controller and the movable mirror, wherein the control module and the scan-head communicate over Ethernet. In particular, such design may be useful when eye surgery requires a surgeon to use the scan-head close to the area being operated, while the control module may be arranged further away from the patient and the surgeon.

According to a fourth aspect, use of the system of the first aspect for optical coherence tomography (OCT) is disclosed.

The above aspects, accompanying claims, and/or examples disclosed herein above and later below may be suitably combined with each other as would be apparent to anyone of ordinary skill in the art.

Additional features and advantages are disclosed in the following description, claims, and drawings, and it may be readily apparent to combine such features disclosed in the context of one aspect or embodiment above with those disclosed in the description someone of ordinary skilled in the art.

### DESCRIPTION OF THE ACCOMPANYING DRAWINGS

Some embodiments of the disclosed technology will now be described by way of example only and with reference to the accompanying drawings in which:
Figure 1 shows schematically a system for imaging a sample according to one embodiment of the present disclosure;
Figure 2 shows schematically an optical coherence tomography scanner for imaging a sample according to one embodiment of the present disclosure;
Figure 3 shows schematically control units of a system for imaging a sample according to one embodiment of the present disclosure;
Figure 4 shows a method for controlling a system for imaging a sample according to one embodiment of the present disclosure;
Figure 5 shows a control logic of the second controller according to one embodiment of the present disclosure.
Figure 6 shows a control logic of the first controller according to one embodiment of the present disclosure.
Figure 7 shows a light source failsafe logic according to one embodiment of the present disclosure.

### DETAILED DESCRIPTION

The detailed description set forth below provides examples of embodiments of the disclosed technology which are explained in sufficient detail to enable those skilled in the art to put the disclosed technology into practice.

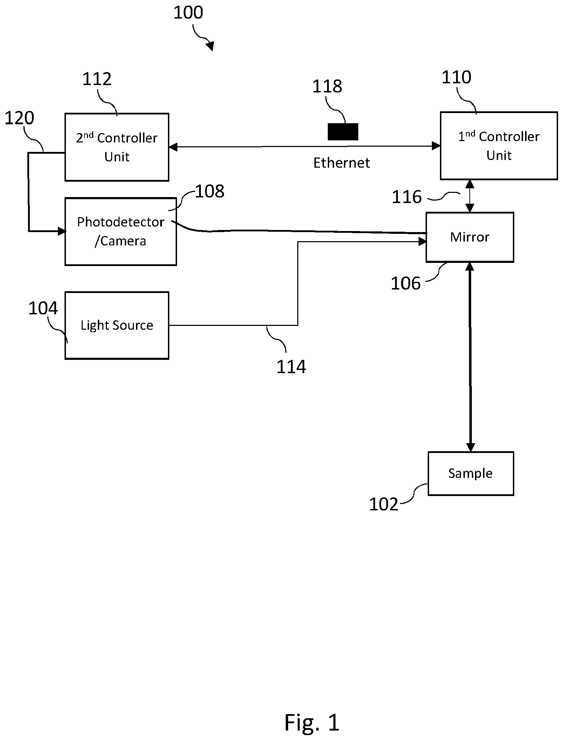

Figure 1 schematically shows the principles of operation of an exemplary system 100 for imaging a sample 102 which comprises some examples of embodiments of the disclosed technology. The system 100 comprises a light source 104, a movable mirror 106, a photodetector 108, a first control unit 110, and a second control unit 112. The light source 104 is configured to generate a light beam 114 and to direct the light beam to the movable mirror which is further configured to deflect the light beam onto the sample 102. The photodetector 108 is configured to detect light reflected from the sample. The sample may reflect light onto the mirror 108, which may further redirect reflected light onto the photodetector 108. The first control unit is communicatively connected to the movable mirror, preferably via an electrical connection link 116, and configured to steer the movable mirror according to position coordinates. The position coordinates may pertain to a tilt angle of the movable mirror. The second control unit 112 is configured to generate a data packet 118 comprising the position coordinates and to send the generated data packet 118 to the first control unit 110. The second control unit is further configured to generate trigger signals 120 for the photodetector 108. The trigger signals 120 are configured to trigger the photodetector to capture images corresponding to the position coordinates of the movable mirror. The first control unit and the second control unit are configured to communicate over Ethernet such that synchronization between the steering of the movable mirror and the triggering of the photodetector is enabled.

It will be apparent that the system 100 is illustrated in Figure 1 schematically and is not drawn to scale. The position of various components and their relative sizes of the system 100 shown in Figure 1 do not necessarily reflect their real or relative positions or sizes in example embodiments of the disclosed technology.

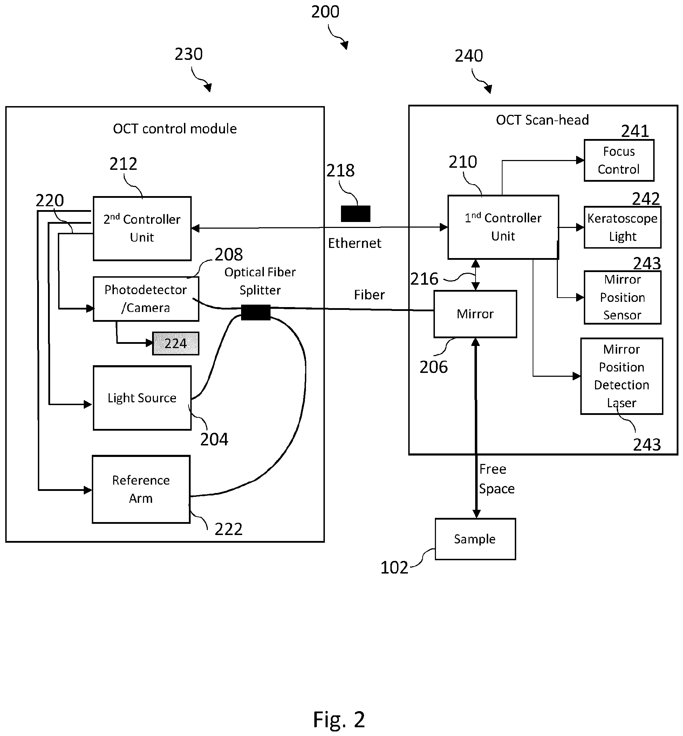

Figure 2 schematically shows the principles of operation of an exemplary optical coherence tomography scanner 200 for imaging a sample 102 which comprises some examples of embodiments of the disclosed technology. The scanner 200 comprises a light source 204, a movable mirror 206, a photodetector 208 (such as a photodiode, an array of photodiodes, a camera, etc.), a first control unit 210, a second control unit 212, and an image processor 224. The light source is configured to generate a light beam and to direct the light beam to the movable mirror which is further configured to deflect the light beam onto the sample. The photodetector is configured to detect light reflected from the sample and to send the detected signal to the image processor configured to generate an image of the sample. The first control unit is communicatively connected to the movable mirror and configured to steer the movable mirror according to position coordinates, the position coordinates preferably pertaining to a tilt angle of the movable mirror. The second control unit is configured to generate a data packet comprising the position coordinates and sending the data packet to the first control unit and the second control unit being further configured to generate trigger signals for the photodetector, the trigger signals being configured to trigger the photodetector to capture images corresponding to the position coordinates of the movable mirror. The first control unit and the second control unit are configured to communicate over Ethernet such that synchronization between the steering of the movable mirror and the triggering of the photodetector is enabled.

The scanner 200 may further comprise a reference arm 222. The reference arm 222 may be fiber-based and/or free-space based and typically guides a beam used for feedback on the movable mirror which is reflected in a different optical plane from that used by the OCT scanning beam. The scanner may include a splitter/coupler226, such as an optical fiber-based coupler, for coupling light from the reference arm 222 and from the light source 204, and to directing it onto the movable mirror 206, as well as for coupling light reflected from the sample into the photodetector 208.

In some embodiments, the scanner may comprise an OCT control module 230 and an OCT scan-head 240. The OCT control module 230 may include the second control unit 212, the photodetector 208, the light source 204, the reference arm 222, the image processor 224, and the optical coupler. The OCT control module may be remotely located from the OCT scan-head, as various requirements on placement and size of the scan-head may be imposed. In such implementations of the OCT scanner it may be beneficial to connect the OCT control module and the scan-head over Ethernet to ensure real-time communication between the two units. Ethernet connection can also enable real-time synchronization between the two modules, in particular synchronization between the steering of the movable mirror 206 and the triggering of the photodetector 208.

The scan-head 240 may include the first control unit 210 and the movable mirror 206. Additionally, the scan-head may include further control units relevant for the mirror positioning, such as a focus control unit 241, keratoscope light 242, mirror position sensor 243, and mirror position detection sensor 244, all being in communication with the first control unit 210.

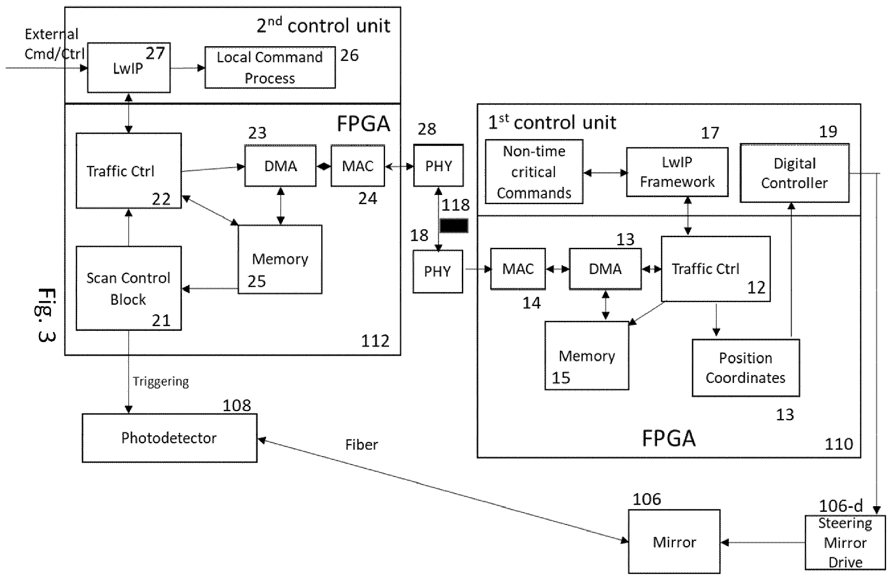

Figure 3 shows schematically control units 110 and 112 of a system for imaging a sample according to embodiments of the present disclosure. In particular, a block diagram for the control units 110, 112 is shown. Both the first control unit 110 and the second control unit 112 may be implemented as FPGAs. The second control unit 112 may comprise of a scan-control block 21. The scan-control block 21 may be configured to generate triggers to both the photodetector 108 and to a traffic-controller block 22. The traffic-controller block may be configured for forwarding data packets 118 to the first control unit 110. The scan-control block 21 may be equipped with a configurable timer (not shown). The timer may be configured to generate triggers based on the desired photodetector frame rate. These triggers initiate fetching of the position coordinates that are passed on to the traffic controller block 22. The position coordinates may be fetched from a memory 25. The traffic controller 22 may place the position coordinates in a predefined data packet, such as a UDP data packet. The traffic controller 22 may return the position coordinates, together with the triggers to the memory 25. The traffic control block 22 may initiate the DMA 23 to transfer the data packet 118 to the first control unit 110. The transfer of the data packet 118 may be assisted by MACs 14, 24 that may be comprised in both the first and second control unit. Ethernet physical layer transistors (PHYs) 18, 28 may be in communication with the MACs of the first and second controller. If there are commands/controls that need to be sent to the first control unit 110, a request from an LwIP 27 may be issued to the traffic controller 22 to forward the data packet 118 (comprising position coordinates) to the first controller 110 interleaved with the required commands/controls.

The first control unit 110 may has a similar configuration on the connection side as the second control unit 110, with e.g. MAC 14, DMA 13, and a traffic controller 12. The first traffic controller 12 may be configured to evaluate the incoming packet 118, such as a packet header to first ensure the protocol type and header parameters (e.g. UDP header parameters). The first traffic controller 12 may then filter out the position coordinates and place the new position coordinates in a memory of a digital controller 19. The digital controller 19 may be configured to control steering of the mirrors 106 by sending the position coordinates to a steering mirror drive 106-d. Other commands/controls may be passed on to a microcontroller lightweight IP (LwlP) driver 17 for upper software processing. These commands/controls are typically non-time critical and may include optical actuator controls, mirror controller runtime parameters, mirror controller performance monitoring, firmware update data, auxiliary light source control, such as keratoscope, and controller fault and status monitoring. These commands/controls may be sent separately from the data packet 118 comprising time-critical position coordinates.

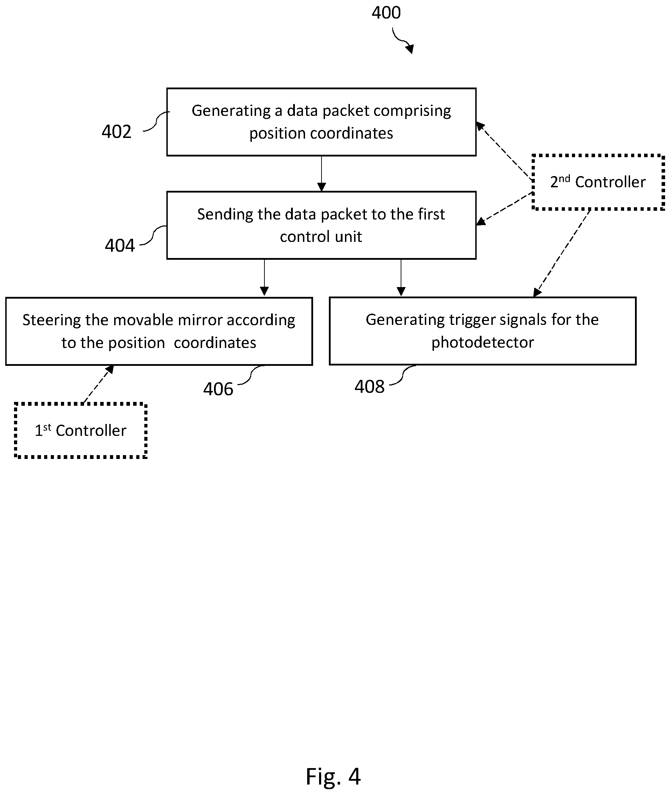

Figure 4 shows a method 400 for controlling a system for imaging a sample according to one embodiment of the present disclosure. The system may be the system 100 as described in connection to Figure 1 or the scanner 200 as described in connection to Figure 2. The method 400 comprises the steps of generating 402, at the second control unit, a data packet comprising position coordinates; sending 404, by the second control unit over Ethernet, the data packet to the first control unit; steering 406, by the first control unit, the movable mirror according to the position coordinates; simultaneously generating 408, at the second control unit, trigger signals for the photodetector such that synchronization between the steering of the mirrors and the triggering of the photodetector is enabled, the trigger signals being configured to trigger the photodetector to capture images corresponding to the position coordinates of the movable mirror.

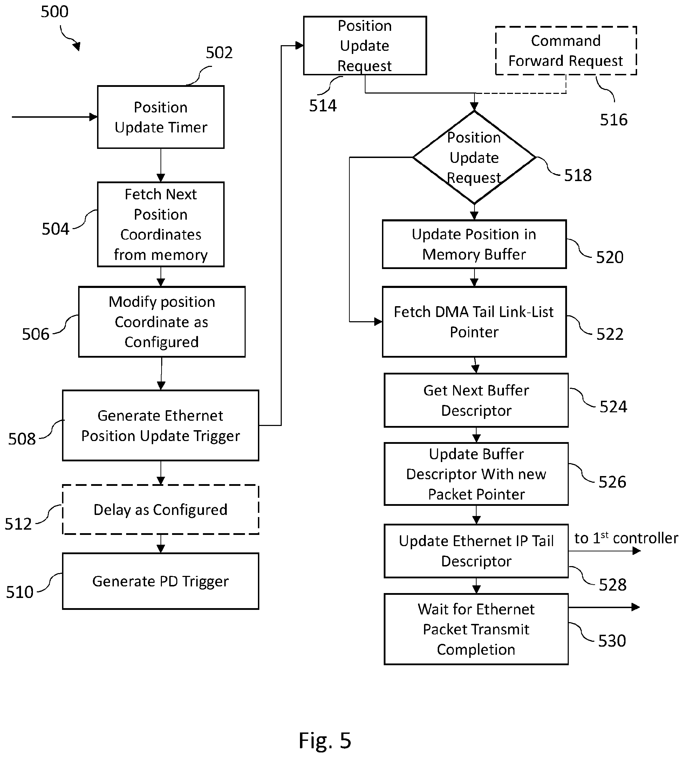

Figure 5 shows control logic 500 of the second controller according to one embodiment of the present disclosure. The traffic controller of the second control unit may comprise a position timer that may initiate a new synchronization session in which new position coordinates are communicated to the movable mirror while the photodetector is simultaneously triggered. The position timer initiates the update process (step 502), that results in the scan-controller block to fetch the next position coordinates (step 504) and modify previous position coordinates accordingly (step 506). The position coordinates may be fetched from a memory of the second controller. Once the position coordinates are updated in the scan-controller block, the scan-controller block may generate a position update trigger (step 508). The scan-controller block then generates a triggering signal for the photodetector (step 510). Optionally, a preconfigured delay may be added to the triggering signal (step 512).

Upon generation of the position update trigger (step 508) the scan-controller generates a position update request to the traffic controller (step 514). In some embodiments, the scan-control block may be configured to modify the position coordinates on the basis of configuration parameters. Modification of the position coordinates may include offset, scaling, rotation, and/or distortion corrections. The configuration parameters may relate to a delay in communication between the first and second control unit, to various delays that may exist in the imaging system, or similar. If the position coordinates are to be modified, the scan control block may send a command request to the traffic controller for updating the position coordinates. Upon modifying the position coordinates, the scan-control block may then generate a new position update request to the traffic-controller (step 518).

The traffic controller may then copy the new position coordinates into a predefined (e.g. UDP) packet buffer (step 520) and update the (UDP) header fields as required. The traffic controller then fetches the DMA tail link-list pointer (step 522), identifies the next available buffer descriptor (step 524), updates the buffer descriptor with the new packet pointer (step 526) to the (UDP) data packet followed by updating the Ethernet block tail buffer descriptor pointer (step 528). This signals the Ethernet block at the second controller to begin transmission of the data packet to the first controller. The traffic controller may also be configured to receive information on completion of the data packet transmission (step 530).

In some embodiments, the control/command module of the second controller may initiate command forwarding (step 516) by signalling the traffic controller block. The command may be placed in a (UDP) frame packet, and the memory address may be passed on to the traffic controller. The traffic controller may follow similar sequence of steps to place the (UDP) frame packet in the next available buffer descriptor for the Ethernet block to process. The frame packet with control/command signals may be placed in between the position update intervals, thereby not affecting the position update timing. Alternatively, the frame packet with control/command signals may be interleaved with the position update intervals.

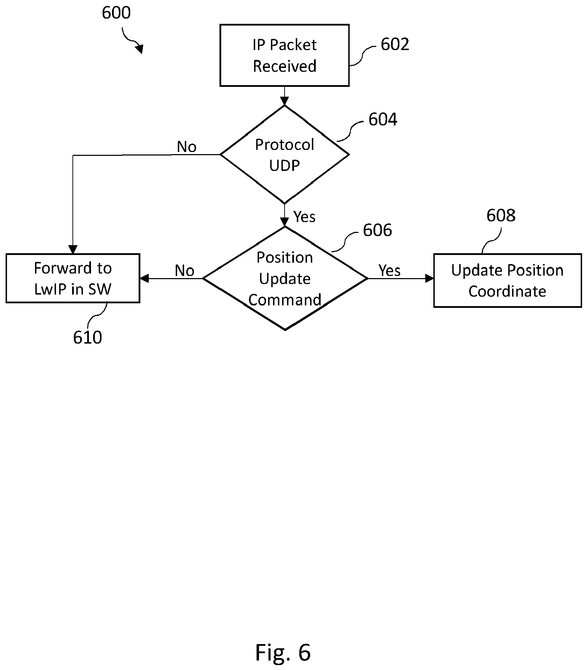

Figure 6 shows control logic 600 of the first controller according to one embodiment of the present disclosure. On the first controller side (e.g. scan-head side), the traffic controller of the first controller evaluates the incoming packet header (step 602) to first identify the protocol type and (UDP) header parameters (step 604). If the receiver packet comprises a header with position coordinates, the traffic controller filters out the position coordinates (step 606) and places the new position coordinates in the digital controller designated memory position (step 608) which will then drive the mirror driver accordingly. If the received packet does not comprise a header with position coordinates, the traffic controller will pass the received packet comprising other commands on to a microcontroller LwlP driver for upper software processing (step 610).

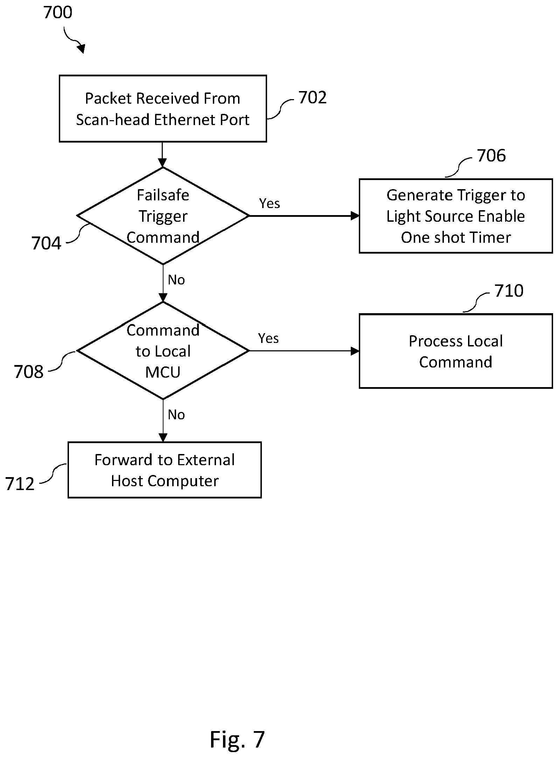

Figure 7 shows a light source failsafe logic 700 according to one embodiment of the present disclosure. This failsafe mechanism may be used to ensure that the light source is shut down if the first controller (e.g. scan-head of OCT scanner) is not active or malfunctions and/or communication between the first and the second control unit is lost (e.g. communication between the scan-head and the OCT control engine). The first controller (e.g. scan-head) may send out periodic "scan-active" commands back to the second controller (OCT control engine) (step 702). Namely, the first controller has an independent process that monitors the motion of the mirror and sends out periodic scan-active status command back to the second controller. These commands are filtered based on the packet header information and monitored by the second controller, which resets the light source shutdown timer whenever the command is received (step 706). If the command fails to arrive before the expiration of the light-source timer, the light source is disabled. This will prevent a static beam to stay on the target sample, which can lead to an unsafe condition for the patient under surgery.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, steps, operations, elements, and/or components, but do not preclude the presence or addition of one or more other features, integers, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

As used herein the term "and/or" includes any and all combinations of one or more of the associated listed items and may be abbreviated as "f".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

It is to be understood that the present disclosure is not limited to the aspects described above and illustrated in the drawings; rather, the skilled person will recognize that many changes and modifications may be made within the scope of the present disclosure and appended claims. In the drawings and specification, there have been disclosed aspects for purposes of illustration only and not for purposes of limitation, the scope of the inventive concepts being set forth in the following claims.

## Claims

1. A system for imaging a sample, the system comprising a light source, a movable mirror, a photodetector, a first control unit and a second control unit,
the light source being configured to generate a light beam and to direct the light beam to the movable mirror which is further configured to deflect the light beam onto the sample,
the photodetector being configured to detect light reflected from the sample,
the first control unit being communicatively connected to the movable mirror and configured to steer the movable mirror according to position coordinates,
the second control unit being configured to generate a data packet comprising the position coordinates and sending the data packet to the first control unit, the second control unit being further configured to generate trigger signals for the photodetector, the trigger signals being configured to trigger the photodetector to capture images corresponding to each of the position coordinates of the movable mirror,
wherein the first control unit and the second control unit are configured to communicate over Ethernet such that synchronization between the steering of the movable mirror and the triggering of the photodetector is enabled.

2. The system of claim 1, wherein the first control unit and the second control unit are further configured to exchange one or more command/control signals over Ethernet, the commands/control signals being related to the system operation.

3. The system according to claim 1 or 2, wherein the first control unit and the second control unit each comprise a field-programmable gate array comprising a position update module and a command module, the position-update module being configured to process data related to the position coordinates and the trigger signals, the command module being configured to process the commands/control signals.

4. The system according to any of the preceding claims, wherein the Ethernet communication between the first and the second control unit is implemented over an Internet Protocol.

5. The system according to any of the preceding claims, wherein the second control unit comprises a scan-control block configured to generate the trigger signal to the photodetector and provide the position coordinates to a traffic-control block, the traffic-control block being configured for sending the data packet comprising the position coordinates and/or command/control requests to the first control unit.

6. The system according to any of the preceding claims, wherein the trigger signals are based on a predetermined photodetector frame rate.

7. The system according to claims 2-6, wherein the data packet further comprises the one or more commands/control signals.

8. The system of claims 5-7, wherein the scan-control block is further configured to modify the position coordinates on the basis of configuration parameters.

9. The system of claim 8, wherein the traffic-control block is configured to receive the modified position coordinates from the scan-control block and to update the data packet with the modified position coordinates.

10. The system according to any of the preceding claims, wherein the first control unit upon receiving the data packet with the position coordinates is configured to retrieve the position coordinates from the data packet and to steer the movable mirror accordingly, and/or to derive the one or more command/control signals for further processing.

11. The system according to any of the preceding claims 2-10, wherein the one or more command/control signals comprise a failsafe command configured to shut-down the light source if the first controller is not active or malfunctions and/or communication between the first and the second control unit is lost.

12. A method for controlling a system for imaging a sample, the system comprising a light source, a movable mirror, a photodetector, a first control unit, and a second control unit, the light source being configured to generate a light beam and to direct the light beam to the movable mirror which is further configured to deflect the light beam onto the sample, the photodetector being configured to detect light reflected from the sample, the first control unit being communicatively connected to the movable mirror, the second control unit being communicatively connected to the photodetector, and the first control unit and the second control unit being configured to communicate over Ethernet,
the method comprising the steps of:
- generating, at the second control unit, a data packet comprising position coordinates,
- sending, by the second control unit over Ethernet, the data packet to the first control unit,
- steering, by the first control unit, the movable mirror according to the position coordinates,
- generating, at the second control unit, trigger signals for the photodetector such that synchronization between the steering of the mirrors and the triggering of the photodetector is enabled, the trigger signals being configured to trigger the photodetector to capture images corresponding to the position coordinates of the movable mirror.

13. An optical coherence tomography scanner for imaging a sample, the scanner comprising a light source, a movable mirror, a photodetector, a first control unit, a second control unit, and an image processor,
the light source being configured to generate a light beam and to direct the light beam to the movable mirror which is further configured to deflect the light beam onto the sample,
the photodetector being configured to detect light reflected from the sample and to send the detected signal to the image processor configured to generate an image of the sample,
the first control unit being communicatively connected to the movable mirror and configured to steer the movable mirror according to position coordinates,
the second control unit being configured to generate a data packet comprising the set of position coordinates and sending the data packet to the first control unit and the second control unit being further configured to generate trigger signals for the photodetector, the trigger signals being configured to trigger the photodetector to capture images corresponding to the position coordinates of the movable mirror,
wherein the first control unit and the second control unit are configured to communicate over Ethernet such that synchronization between the steering of the movable mirror and the triggering of the photodetector is enabled.

14. Use of the system of claims 1-11 for optical coherence tomography.
